# EUROPEAN PATENT APPLICATION

(11) **EP 4 464 360 A1**
(43) Date of publication of application: **20.11.2024**
(21) Application number: 24174506.6
(22) Date of filing: 07.05.2024
(51) Int. Cl.: A61M 25/00, A61M 25/01, A61M 25/06, A61B 18/14

(54) **TELESCOPIC SHEATH**

(30) Priority: 08.05.2023 US 202318144808
(71) Applicant: Biosense Webster (Israel) Ltd., 2066717 Yokneam (IL)
(72) Inventor: GOVARI, Assaf, 2066717 Yokneam (IL)
(74) Representative: Carpmaels & Ransford LLP

(57) **Abstract**

A telescopic sheath (14) includes an outer sleeve (40) extending from a proximal end (44) to a distal end (46), wherein the distal end is configured for being inserted into a body, an inner sleeve (42) that telescopically extends proximally from the proximal end of the outer sleeve, a fluid seal (48), which is disposed between the inner sleeve and outer sleeve and is configured to prevent back bleeding through a space between the inner sleeve and outer sleeve, and a lock (52), configured to fix a position of the inner sleeve with respect to the outer sleeve. The sheath is configured to guide an intrabody catheter (16) by virtue of the catheter passing from a proximal end of the inner sleeve to the distal end of the outer sleeve.

## Description

### FIELD OF THE INVENTION

The present disclosure is related generally to the field of medical devices, and particularly to sheaths for the guidance of intrabody catheters.

### BACKGROUND OF THE INVENTION

In some medical procedures, such as cardiac mapping and/or ablation procedures, a sheath, which is percutaneously inserted into the vasculature of a patient, is used to guide the insertion and subsequent navigation of an intrabody catheter.

### BRIEF DESCRIPTION OF THE DRAWINGS

The present disclosure will be more fully understood from the following detailed description of examples thereof, taken together with the drawings, in which:
Fig. 1 is a schematic illustration of an example electrophysiology mapping and ablation system, in accordance with some examples of the present disclosure; and
Figs. 2A-B and 3A-B are schematic illustrations of a telescopic sheath, in accordance with some examples of the present disclosure.

### DETAILED DESCRIPTION

### OVERVIEW

A sheath for guiding an intrabody catheter during a procedure must be long enough to reach the portion of anatomy on which the procedure is to be performed. For example, a longer sheath may be required for a procedure on the left ventricle, relative to a procedure on the right ventricle. The minimal required length may also vary as a function of anatomical differences between patients.

On the other hand, the sheath must not be too long so as to compromise the physician's ability to maneuver the sheath or catheter. As a result, there is no single sheath length that is suitable for all procedures.

Hence, conventionally, the physician is provided with multiple sheaths of different lengths from which to select. However, sometimes the physician discovers, after inserting the selected sheath into the patient, that the length of the selected sheath is unsuitable. As a result, the physician must exchange the sheath for a different sheath, thus complicating, and increasing the duration of, the procedure.

To address this challenge, examples of the present disclosure provide a telescopic sheath having an adjustable length. The sheath comprises an outer sleeve and a shorter, narrower inner sleeve that telescopically extends proximally from the proximal end of the outer sleeve. To adjust the length of the sheath, the physician may simply slide the two sleeves relative to one another.

The telescopic sheath further comprises a fluid seal between the two sleeves, which advantageously inhibits blood from flowing proximally through the proximal end of the outer sleeve.

In addition, the telescopic sheath comprises a lock, which locks the relative positions of the two sleeves, and thus, fixes the length of the sheath following the adjustment thereof.

### SYSTEM DESCRIPTION

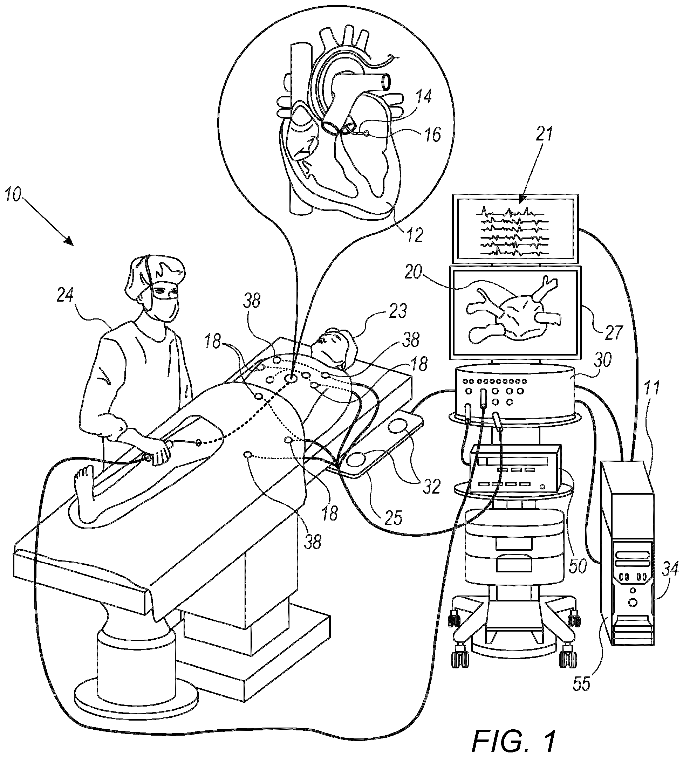

Reference is initially made to Fig. 1, which is a schematic illustration of an example electrophysiology mapping and ablation system 10, in accordance with some examples of the present disclosure. One commercial product embodying elements of system 10 is available as the CARTO^{™} 3 System, available from Biosense Webster, Inc., 31A Technology Drive, Irvine, CA 92618.

System 10 comprises at least one intrabody catheter 16, which comprises electrodes for sensing intracardiac electrogram (IEGM) signals and/or ablating cardiac tissue. Catheter 16 is percutaneously inserted by a physician 24 into the body of a patient 23. Following the insertion of the catheter, physician 24 navigates the catheter through the vascular system of patient 23 into a chamber or vascular structure of the patient's heart 12. In some examples, a telescopic sheath 14, which is described with reference to the subsequent figures, guides the catheter during the insertion and navigation thereof.

Typically, catheter 16 comprises a tracking sensor for tracking the three-dimensional (3D) location and orientation of the distal end of the catheter. Typically, the tracking sensor comprises three magnetic coils. A location pad 25, which comprises a plurality of magnetic coils 32 configured to generate a magnetic field in a predefined working volume, is positioned near (e.g., underneath) patient 23. Based on signals induced in the coils by the magnetic field, the location and orientation of the distal end of the catheter are tracked. Details of such magnetic-based tracking are described in U.S. Patent Nos. 5,5391,199, 5,443,489, 5,558,091, 6,172,499, 6,239,724, 6,332,089, 6,484,118, 6,618,612, 6,690,963, 6,788,967, and 6,892,091.

Typically, system 10 further comprises one or more electrode patches 38 configured to contact the skin of patient 23. Patches 38 may establish a location reference for location pad 25. Additionally, electrical current from the electrodes on catheter 16 may be sensed at patches 38, and the location of each electrode may be triangulated in response thereto. This location information may be combined with the information derived from the magnetic-based tracking described above so as to increase the precision of the tracking. Details of such impedance-based tracking technology are described in US Patent Nos. 7,536,218, 7,756,576, 7,848,787, 7,869,865, and 8,456,182.

System 10 further comprises a recorder 11 and a display 27. Recorder 11 is configured to record electrocardiographic (ECG) signals 21 acquired by body-surface ECG electrodes 18 and/or IEGM signals acquired by catheter 16, and optionally, to display these signals on display 27. Recorder 11 may also be configured to pace heart 12 and/or may be electrically connected to a standalone pacer.

System 10 further comprises an ablation-energy generator 50 configured to conduct ablative energy to any ablation electrodes at the distal end of catheter 16. Energy produced by ablation-energy generator 50 may include, but is not limited to, radiofrequency (RF) energy and/or PFA energy, including monopolar or bipolar high-voltage direct-current (DC) pulses for effecting IRE, or combinations thereof. Ablation-energy generator 50 comprises energy-generating circuitry configured to produce the energy, along with a controller configured to control the circuitry and, optionally, perform other computational functions.

System 10 further comprises a workstation 55 comprising a processor 34, a volatile memory and/or non-volatile memory that may store appropriate software instructions and/or data, and a user interface. Processor 34 may be configured to perform multiple functions, including, for example, (1) mapping the endocardial anatomy of heart 12 in 3D and rendering the resulting anatomical map 20 for display on display 27, (2) displaying, on display 27, activation sequences and/or other data compiled from ECG signals 21 in representative visual indicia or imagery superimposed on anatomical map 20, (3) displaying the real-time location and orientation of one or more catheters within the body of patient 23, and (4) displaying sites of interest, such as sites at which ablation energy has been applied.

System 10 further comprises a patient interface unit (PIU) 30, which is configured to establish electrical communication between power supplies, workstation 55, and the electrophysiological equipment belonging to the system. The electrophysiological equipment may comprise, for example, one or more catheters, location pad 25, ECG electrodes 18, electrode patches 38, ablation-energy generator 50, and/or recorder 11. Typically, PIU 30 further comprises another processor configured to compute of location and orientation of each of the catheters and to perform any relevant computations based on ECG signals 21.

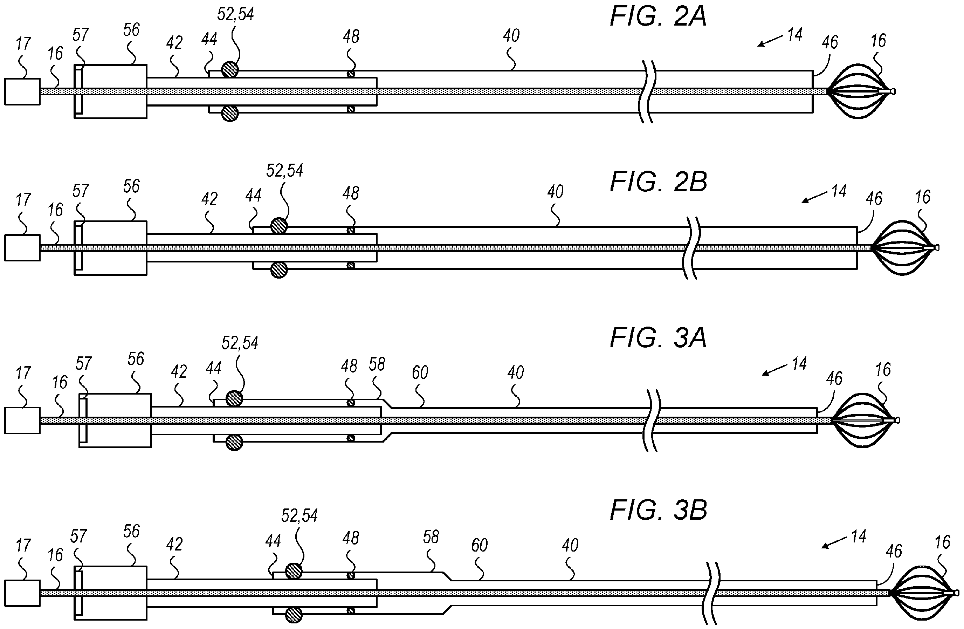

Reference is now made to Figs. 2A-B, which are schematic illustrations of telescopic sheath 14, in accordance with some examples of the present disclosure.

Telescopic sheath 14 comprises an outer sleeve 40, which extends from a proximal end 44 to a distal end 46. Distal end 46 is configured for insertion into the body of patient 23 (Fig. 1).

Telescopic sheath 14 further comprises an inner sleeve 42 disposed within outer sleeve 40 and telescopically extending proximally from proximal end 44. In other words, inner sleeve 42 extends proximally from the outer sleeve, with the length of the proximally-extending portion of the inner sleeve - and thus, the total length of sheath 14 - being adjustable. For example, to lengthen the sheath, the outer sleeve may be advanced distally while the inner sleeve is held in place or is retracted proximally. Conversely, to shorten the sheath, the outer sleeve may be retracted proximally while the inner sleeve is held in place or is advanced distally.

For example, Fig. 2A shows sheath 14 in a less extended configuration, in which inner sleeve 42 is mostly within outer sleeve 40. In contrast, Fig. 2B shows sheath 14 in a more extended configuration, in which inner sleeve 42 is mostly proximal to outer sleeve 40.

Sheath 14 is configured to guide catheter 16 by virtue of the catheter passing from the proximal end of the inner sleeve to distal end 46.

Sheath 14 further comprises a fluid seal 48, e.g., an O-ring, which is disposed between the inner sleeve and outer sleeve and is configured to prevent back bleeding through the space between the inner sleeve and outer sleeve, thereby isolating the interior of the sheath from the outside environment. In other words, fluid seal 48 is configured to inhibit the flow of fluid across the fluid seal, such that the fluid seal prevents blood of the patient, which may collect within outer sleeve 40 distally to the seal, from reaching proximal end 44. (Fluid seal 48 is shown in cross-section in Figs. 2A-B.)

In some examples, fluid seal 48 is coupled to the inner wall of outer sleeve 40. In other examples, as shown in Figs. 2A-B and 3A-B, fluid seal 48 is coupled to the outer wall of inner sleeve 42.

In general, fluid seal 48 is selected or constructed so as to effectively prevent back bleeding while also generating relatively little friction while the length of the sheath is adjusted.

In some examples, the distal end of the inner sleeve comprises a stopper, which prevents the distal end of the inner sleeve from sliding proximally past fluid seal 48.

Sheath 14 further comprises a lock 52 configured to fix the position of the inner sleeve with respect to the outer sleeve, i.e., to fix the length of the proximally-extending portion of the inner sleeve. Thus, after adjusting the sheath to the desired length, physician 24 (Fig. 1) may fix the length of the sheath.

In general, lock 52 may be locked or unlocked as needed, i.e., lock 52 is configured to transition, as many times as needed, between a locked state and an unlocked state.

In some examples, as illustrated in Figs. 2A-B, lock 52 comprises a ring 54 configured to fix the position of the inner sleeve by clamping the outer sleeve against the inner sleeve. (Ring 54 is shown in cross-section in Figs. 2A-B.) In such examples, outer sleeve 40 may be shaped to define an indentation, and ring 54 may fit within the indentation.

Typically, lock 52 is configured to transfer, to the outer sleeve, torque that is applied to the inner sleeve. In other words, the lock rotationally locks the two sleeves with respect to one another, such that the physician may rotate the outer sleeve simply by rotating the inner sleeve.

Typically, sheath 14 further comprises a handle 56 coupled to the proximal end of the inner sleeve. The physician may grip handle 56 while rotating, or adjusting the length of, the sheath.

Typically, sheath 14 further comprises a valve 57 coupled to the proximal end of the inner sleeve and configured to prevent back bleeding through the proximal end of the inner sleeve, thereby isolating the interior of the sheath from the outside environment. In other words, valve 57 is configured to inhibit the proximal flow of fluid across the valve, such that the valve prevents blood of the patient, which may collect within inner sleeve 42, from exiting the sheath. In such examples, catheter 16 is inserted into sheath 14 through valve 57.

For example, handle 56 may comprise valve 57. As a specific example, valve 57 may be disposed at the proximal end of the handle.

In some examples, as shown in Figs. 2A-B, the width of outer sleeve 40 is constant. For example, assuming that each of the sleeves has a circular cross-section, the circumference of the inner sleeve may be between 8.5 and 12 Fr, and the circumference of the outer sleeve may be around 1 Fr greater than that of the inner sleeve.

To facilitate manipulating the catheter, the physician may grip a handle 17 at the proximal end of the catheter.

Reference is now made to Figs. 3A-B, which are schematic illustrations of telescopic sheath 14, in accordance with some examples of the present disclosure. Fig. 3A shows a less extended configuration of sheath 14, while Fig. 3B shows a more extended configuration.

As opposed to the example shown in Figs. 2A-B, Figs. 3A-B show an example in which inner sleeve 42 is configured to telescopically slide within a proximal portion 58 of outer sleeve 40, and the outer sleeve narrows distally to proximal portion 58. Thus, advantageously, the length of the sheath is adjustable as in Figs. 2A-B, yet the sheath may be more maneuverable and more capable of penetrating narrower lumens, relative to Figs. 2A-B.

For example, the outer sleeve may narrow (over any suitable length of the outer sleeve) to the circumference of the inner sleeve, such that the outer sleeve comprises two main portions: proximal portion 58, the circumference of which is greater than (e.g., around 1 Fr greater than) the circumference of the inner sleeve as in Figs. 2A-B, and a more distal portion 60, the circumference of which is identical to the circumference of the inner sleeve.

In such examples, the sheath may be minimally extended when the distal end of the inner sleeve is at the distal end of proximal portion 58. As shown in Figs. 3A-B, the length of proximal portion 58 may be less than the length of the inner sleeve.

Typically, to provide sufficient adjustability while limiting manufacturing costs, the length of the inner sleeve is less than 50 cm, e.g., 10-50 cm. Alternatively or additionally, the outer sleeve may be at least three times as long as the inner sleeve.

As a purely illustrative example, the length of the outer sleeve may be around 130 cm and the length of the inner sleeve may be around 30 cm. Thus, assuming the inner sleeve is fully insertable into, and extendible from, the outer sleeve (as in Figs. 2A-B), the total length of the sheath may vary between around 130 cm and around 160 cm. Alternatively, in case the inner sleeve is not fully insertable into the outer sleeve (as in Figs. 3A-B) or is not fully extendible from the outer sleeve, the range of lengths may be different, e.g., 140-160 cm or 130-150 cm.

### EXAMPLES

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

A telescopic sheath (14) includes an outer sleeve (40) extending from a proximal end (44) to a distal end (46), the distal end being configured for being inserted into a body. The telescopic sheath further includes an inner sleeve (42) that telescopically extends proximally from the proximal end of the outer sleeve. The telescopic sheath further includes a fluid seal (48), which is disposed between the inner sleeve and outer sleeve and is configured to prevent back bleeding through a space between the inner sleeve and outer sleeve, and a lock (52) configured to fix a position of the inner sleeve with respect to the outer sleeve. The sheath is configured to guide an intrabody catheter (16) by virtue of the catheter passing from a proximal end of the inner sleeve to the distal end of the outer sleeve.

### Example 2

The telescopic sheath (14) according to Example 1, further including a handle (56) coupled to a proximal end of the inner sleeve (42).

### Example 3

The telescopic sheath (14) according to any one of Examples 1-2, wherein the inner sleeve (42) is configured to telescopically slide within a proximal portion (58) of the outer sleeve (40), and wherein the outer sleeve narrows distally to the proximal portion of the outer sleeve.

### Example 4

The telescopic sheath (14) according to Example 3, wherein the outer sleeve (40) narrows to a circumference of the inner sleeve (42).

### Example 5

The telescopic sheath (14) according to any one of Examples 1-4, wherein the lock (52) includes a ring (54) configured to fix the position of the inner sleeve (42) by clamping the outer sleeve (40) against the inner sleeve.

### Example 6

The telescopic sheath (14) according to any one of Examples 1-5, further including a valve (57) coupled to a proximal end of the inner sleeve (42) and configured to prevent back bleeding through the proximal end of the inner sleeve.

### Example 7

The telescopic sheath (14) according to Example 6, further including a handle (56) coupled to the proximal end of the inner sleeve (42), wherein the handle includes the valve (57).

### Example 8

The telescopic sheath (14) according to any one of Examples 1-7, wherein the lock (52) is configured to transfer, to the outer sleeve (40), torque that is applied to the inner sleeve (42).

### Example 9

The telescopic sheath (14) according to any one of Examples 1-8, wherein a length of the inner sleeve (42) is less than 50 cm.

### Example 10

The telescopic sheath (14) according to any one of Examples 1-9, wherein the outer sleeve (40) is at least three times as long as the inner sleeve (42).

### Example 11

A method for manufacturing a telescopic sheath (14) includes inserting an inner sleeve (42) and a fluid seal (48) into an outer sleeve (40) extending from a proximal end (44) to a distal end (46), the distal end being configured for being inserted into a body, such that the inner sleeve telescopically extends proximally from the proximal end of the outer sleeve and the fluid seal is disposed between the inner sleeve and outer sleeve. The fluid seal is configured to prevent back bleeding through a space between the inner sleeve and outer sleeve. The method further includes providing a lock (52) configured to fix a position of the inner sleeve with respect to the outer sleeve.

### Example 12

The method according to Example 11, further including coupling a handle (56) to a proximal end of the inner sleeve (42).

### Example 13

The method according to any one of Examples 11-12, wherein inserting the inner sleeve (42) into the outer sleeve (40) includes inserting the inner sleeve into a proximal portion (58) of the outer sleeve such that the inner sleeve is configured to telescopically slide within the proximal portion of the outer sleeve, and wherein the outer sleeve narrows distally to the proximal portion of the outer sleeve.

### Example 14

The method according to Example 13, wherein the outer sleeve narrows to a circumference of the inner sleeve.

### Example 15

The method according to any one of Examples 11-14, wherein the lock (52) includes a ring (54) configured to fix the position of the inner sleeve (42) by clamping the outer sleeve (40) against the inner sleeve.

### Example 16

The method according to any one of Examples 11-15, further including coupling a valve (57) to a proximal end of the inner sleeve (42), the valve being configured to prevent back bleeding through the proximal end of the inner sleeve.

### Example 17

The method according to Example 16, wherein coupling the valve to the proximal end of the inner sleeve includes coupling the valve to the proximal end of the inner sleeve by coupling a handle, which includes the valve, to the proximal end of the inner sleeve.

### Example 18

The method according to any one of Examples 11-17, wherein the lock is configured to transfer, to the outer sleeve, torque that is applied to the inner sleeve.

### Example 19

The method according to any one of Examples 11-18, wherein a length of the inner sleeve is less than 50 cm.

### Example 20

The method according to any one of Examples 11-19, wherein the outer sleeve is at least three times as long as the inner sleeve.

It will be appreciated by persons skilled in the art that the present disclosure is not limited to what has been particularly shown and described hereinabove. Rather, the scope of the present disclosure includes both combinations and subcombinations of the various features described hereinabove, as well as variations and modifications thereof that are not in the prior art, which would occur to persons skilled in the art upon reading the foregoing description. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. A telescopic sheath (14), comprising:
an outer sleeve (40) extending from a proximal end (44) to a distal end (46), wherein the distal end is configured for being inserted into a body;
an inner sleeve (42) that telescopically extends proximally from the proximal end of the outer sleeve;
a fluid seal (48), which is disposed between the inner sleeve and outer sleeve and is configured to prevent back bleeding through a space between the inner sleeve and outer sleeve; and
a lock (52), configured to fix a position of the inner sleeve with respect to the outer sleeve,
the sheath being configured to guide an intrabody catheter (16) by virtue of the catheter passing from a proximal end of the inner sleeve to the distal end of the outer sleeve.

2. The telescopic sheath (14) according to claim 1, further comprising a handle (56) coupled to a proximal end of the inner sleeve (42).

3. The telescopic sheath (14) according to claim 1 or claim 2, wherein the inner sleeve (42) is configured to telescopically slide within a proximal portion (58) of the outer sleeve (40), and wherein the outer sleeve narrows distally to the proximal portion of the outer sleeve.

4. A method for manufacturing a telescopic sheath (14), the method comprising:
inserting an inner sleeve (42) and a fluid seal (48) into an outer sleeve (40) extending from a proximal end (44) to a distal end (46), wherein the distal end is configured for being inserted into a body, such that the inner sleeve telescopically extends proximally from the proximal end of the outer sleeve and the fluid seal is disposed between the inner sleeve and outer sleeve,
the fluid seal being configured to prevent back bleeding through a space between the inner sleeve and outer sleeve;
and
providing a lock (52) configured to fix a position of the inner sleeve with respect to the outer sleeve.

5. The method according to claim 4, further comprising coupling a handle (56) to a proximal end of the inner sleeve (42).

6. The method according to claim 4 or claim 5, wherein inserting the inner sleeve (42) into the outer sleeve (40) comprises inserting the inner sleeve into a proximal portion (58) of the outer sleeve such that the inner sleeve is configured to telescopically slide within the proximal portion of the outer sleeve, and wherein the outer sleeve narrows distally to the proximal portion of the outer sleeve.

7. The telescopic sheath according to claim 3 or the method according to claim 6, wherein the outer sleeve narrows to a circumference of the inner sleeve.

8. The telescopic sheath according to any of claims 1 to 3 and 7 or the method according to any of claims 4 to 7, wherein the lock (52) includes a ring (54) configured to fix the position of the inner sleeve (42) by clamping the outer sleeve (40) against the inner sleeve.

9. The telescopic sheath (14) according to any of claims 1 to 3, 7 and 8, further comprising a valve coupled to a proximal end of the inner sleeve (42) and configured to prevent back bleeding through the proximal end of the inner sleeve.

10. The telescopic sheath (14) according to claim 9, further comprising a handle (56) coupled to the proximal end of the inner sleeve (42), wherein the handle comprises the valve.

11. The method according to any of claims 4 to 8, further comprising coupling a valve (57) to a proximal end of the inner sleeve (42), the valve being configured to prevent back bleeding through the proximal end of the inner sleeve.

12. The method according to claim 11, wherein coupling the valve to the proximal end of the inner sleeve comprises coupling the valve to the proximal end of the inner sleeve by coupling a handle, which includes the valve, to the proximal end of the inner sleeve.

13. The telescopic sheath according to any of claims 1 to 3 and 7 to 10 or the method according to any of claims 4 to 8, 11 and 12, wherein the lock is configured to transfer, to the outer sleeve, torque that is applied to the inner sleeve.

14. The telescopic sheath according to any of claims 1 to 3, 7 to 10 and 13 or the method according to any of claims 4 to 8 and 11 to 13, wherein a length of the inner sleeve is less than 50 cm.

15. The telescopic sheath according to any of claims 1 to 3, 7 to 10, 13 and 14 or the method according to any of claims 4 to 8 and 11 to 14, wherein the outer sleeve is at least three times as long as the inner sleeve.
